Europäisches Patentamt

European Patent Office

Office européen des brevets

(11) Veröffentlichungsnummer : **0 027 623**
**B1**

# (12) EUROPÄISCHE PATENTSCHRIFT

(45) Veröffentlichungstag der Patentschrift :
**30.03.83**

(21) Anmeldenummer : **80106214.2**

(22) Anmeldetag : **13.10.80**

(51) Int. Cl.³ : **A 01 N 37/22**, A 01 N 65/00,
A 01 N 43/14, A 61 K 31/715,
A 61 K 31/165, A 61 K 35/78

(54) **Präparat zur Bekämpfung der Ansaug-Mastitis und der Pyogenes-Mastitis bei Rindern.**

(30) Priorität : **20.10.79 DE 2942537**

(43) Veröffentlichungstag der Anmeldung :
**29.04.81 Patentblatt 81/17**

(45) Bekanntmachung des Hinweises auf die Patenterteilung : **30.03.83 Patentblatt 83/13**

(84) Benannte Vertragsstaaten :
**AT CH DE GB LI**

(56) Entgegenhaltungen :
**FR A 2 117 727**
**GB A 955 309**
**US A 3 993 777**
**ARZNEIMITTEL-FORSCHUNG ; Band 28 (I), Heft
3, 1978, Aulendorf, DE, A. WACKER : « Antivirale
Wirkung von Pflanzeninhaltsstoffen », Seiten
347-350**

(73) Patentinhaber : **Henkel Kommanditgesellschaft auf
Aktien**
**Postfach 1100 Henkelstrasse 67**
**D-4000 Düsseldorf 1 (DE)**

(72) Erfinder : **Lauermann, Georg, Dr.**
**Burscheider Weg 123**
**D-4021 Metzkausen (DE)**
Erfinder : **Koch, Ferdinand**
**Händelstrasse 36**
**D-4010 Hilden (DE)**

Präparat zur Bekämpfung der Ansaug-Mastitis und der Pyogenes-Mastitis bei Rindern

Seit Jahren besteht im norddeutschen Raum, vor allem in der sommerlichen Weidezeit, das Problem der Übertragung von hochvirulenten Bakterien (Streptococcus pyogenes) durch Fliegen auf die Zitzen von Rindern, insbesondere von heranwachsenden tragenden Jungrindern. Inzwischen tritt diese Krankheit auch in anderen Gebieten auf, wo ebenfalls vor allem tragende Jungrinder infiziert werden. Die Infektionskrankheit, die medikamentös nicht zu behandeln ist, trägt die Bezeichnung « Pyogenes-Mastitis ». Häufig sind Notschlachtungen die Folge der Infektion.

Weiterhin tritt in süddeutschen Ländern eine Unart der hochtragenden Jungrinder immer mehr hervor, nämlich sich gegenseitig zu besaugen. Daraus können schwere Euterentzündungen (Mastitiden) entstehen, die in der Hauptsache durch Corynebakterien verursacht werden, die im Rachenring der Tiere nachzuweisen sind. Ähnlich wie bei der Pyogenes-Mastitis ist hier die Zahl der Notschlachtungen erheblich.

Beide Krankheiten verursachen somit schweren wirtschaftlichen Schaden.

Die Erfindung betrifft ein flüssiges Produkt zur Euterbehandlung, das die Zitzenöffnungen mit einem Film atmungsaktiv abdeckt und durch einen Bitterstoff das Saugen durch andere Tiere sofort nach der Geschmackswahrnehmung unterbricht.

Beansprucht wird demgemäß ein Präparat zur Bekämpfung der Ansaug-Mastitis und der Pyogenes-Mastitis bei Rindern, gekennzeichnet durch folgende Zusammensetzung :

0,2-8 Gew.-% Naringin (Bitterstoff der Grapefruit)
0,05-5 Gew.-% eines Celluloseethers als Verdickungsmittel
10-45 Gew.-% Propylenglykol-1,2
0,002-0,04 Gew.-% Benzyldiethyl-(2,6-xylylcarbamoylmethyl)-ammoniumbenzoat
0-0,5 Gew.-% eines wasserlöslichen Farbstoffes
Rest Wasser

Naringin ist ein Bitterstoff, der aus unreifen Früchten, Blüten und Rinden des Grapefruit-Baumes gewonnen wird.

Als Verdickungsmittel zur Einstellung der Viskosität können handelsübliche Celluloseether eingesetzt werden, z. B. Methylcellulose oder Carboxymethylcellulose. Besonders bevorzugt wird Methylhydroxypropylcellulose. Die Viskosität des Produktes wird zweckmäßig auf einen solchen Wert eingestellt, daß der Auslauf im DIN-Becher Nr. 4 bei 20 °C 25-35 Sekunden beträgt (DIN-Methode 53 211).

Benzyldiethyl-(2,6-xylylcarbamoylmethyl)-ammoniumbenzoat ist ebenfalls ein stark bitter schmeckender Stoff, der auch unter dem Handelsnamen Bitrex[R] bekannt ist.

Der wasserlösliche Farbstoff dient zur Kontrolle des Auftrags und der Haftung des Produktes auf der Zitzenhaut. Vorzugsweise wird Methylenblau eingesetzt.

Die Wirkstofformulierung wird mit Wasser — vorzugsweise destilliertem oder demineralisiertem Wasser — auf 100 Gew.-% aufgefüllt.

Der bittere Geschmack des Präparates ist derartig abschreckend, daß Rinder, die an sich bittere Stoffe bevorzugen, die aufgenommenen Bitterstoffe unter Speichelabsonderung loszuwerden versuchen. Tiere, die den Bitterstoff einmal wahrgenommen haben, schrecken vor neuem Besaugen zurück.

Der Bitterstoff wird auch von Fliegen verabscheut. Fliegende Insekten, die ihren Saugrüssel auf die Zitzenöffnungen aufsetzen, verlassen diesen Platz schnell wieder.

Durch Wasser kann das Präparat abgewaschen werden. Der extrem bittere Geschmack bleibt aber dann, wenn auch gemildert, noch weiter erhalten. Vor dem Anmelken der Tiere, nach der ersten Geburt, kann der Bittergeschmack durch mehrmaliges Abwischen mit feuchten Lappen weitgehend entfernt werden.

Toxikologisch ist das Präparat völlig unbedenklich. Schädliche oder geschmacksverändernde Rückstände in der Milch können bei sachgemäßer Anwendung nicht auftreten. Das Präparat schadet der Entwicklung des juvenilen Euters bei Jungrindern nicht und ist völlig hautverträglich.

Das Präparat wird mit Hilfe eines Dipbechers, wie bei der Zitzendesinfektion nach dem Melken, aufgetragen. Die Aufbringung mit einem Holzspatel, Pinsel, Schwamm oder dgl. bei sehr kleinen Zitzen ist ebenfalls möglich. Der Farbstoff zeigt an, wie weit die Zitze eingetaucht oder bestrichen wurde.

Unter normalen sommerlichen Verhältnissen ist eine Nachbehandlung der Zitzen etwa alle 2 Wochen erforderlich. In regenreicher Zeit empfiehlt sich eine häufigere Kontrolle — z. B. durch Auflegen des feuchten Fingers auf die Zitzenhaut und anschließende Geschmacksprobe — wieweit noch Bitterstoffe vorhanden sind.

Aus der US-A-3 993 777 ist ein Präparat zur Euterbehandlung von melkenden Kühen bekannt, dessen Wirkung auf einem Gehalt an germiciden quaternären Ammoniumverbindungen beruht. Dieses Präparat soll Mastitis-Erkrankungen dadurch verhindern, daß auf die Euterhaut übertragene Erreger abgetötet werden. Das gegenseitige Besaugen der Jungrinder sowie die Belästigung durch Fliegen kann hiermit jedoch nicht unterbunden werden. Aus GB-A-955 309 (bzw. DE-B-1 280 882) sind quaternäre Ammoniumverbindungen mit extrem bitterem Geschmack bekannt, die zur Denaturierung organischer Substanzen,

**0 027 623**

insbesondere Ethanol, verwendet werden. Ein Hinweis auf die erfindungsgemäße Verwendung bestimmter Bitterstoffe wird nicht gegeben.

Ferner ist aus FR-A-2 117 727 bzw. Arzneimittel — Forschung, Band 28 (I), Heft 3 (1978), Seiten 347-350 bekannt, daß Naringinderivate therapeutische Eigenschaften aufweisen bzw. Naringin unter bestimmten Bedingungen gegen Viren wirksam ist. Ein Hinweis auf die erfindungsgemäße Verwendung von Naringin findet sich nicht.

Beispiel

1,0  Gew.-% Naringin
1,0  Gew.-% Methylhydroxypropylcellulose (Culminal MHPC 1 500 [R] der Firma Henkel)
40,0  Gew.-% Propylenglykol-1,2
0,02 Gew.-% Bitrex [R]
0,04 Gew.-% Methylenblau
57,94 Gew.-% Kondenswasser

**Anspruch**

Präparat zur Bekämpfung der Ansaug-Mastitis und der Pyogenes-Mastitis bei Rindern, gekennzeichnet durch folgende Zusammensetzung :

0,2-8 Gew.-% Narigin (Bitterstoff der Grapefruit)
0,05-5 Gew.-% eines Celluloseethers als Verdickungsmittel
10-45 Gew.-% Propylenglykol-1,2
0,002-0,04 Gew.-% Benzyldiethyl-(2,6-xylylcarbamoylmethyl)-ammoniumbenzoat
0-0,5 Gew.-% eines wasserlöslichen Farbstoffes
Rest Wasser

**Claim**

A preparation for controlling suckling-induced mastitis and pyogenic mastitis in beef cattle, characterised by the following composition :

0.2 to 8.0 % by weight of naringin (the bitter component of grapefruit)
0.05 to 5 % by weight of a cellulose ether as thickener
10 to 45 % by weight of 1,2-propylene glycol
0.002 to 0.04 % by weight of benzyldiethyl-(2,6-xylylcarbamoylmethyl)-ammonium benzoate
0 to 0.5 % by weight of a water-soluble dye
remainder water.

**Revendication**

Composition pour la lutte contre la mastite d'allaitement et la mastite des pyogènes chez les bovins, caractérisée par la composition suivante :

0,2-8 % en poids de naringine (principe amer du pamplemousse),
0,05-5 % en poids d'un éther de cellulose comme épaississant,
10-45 % en poids de propylèneglycol-1,2,
0,002-0,04 % en poids de benzoate de benzyldiéthyl-(2,6-xylylcarbamoylméthyl) ammonium,
0-0,5 % en poids d'un colorant soluble dans l'eau
le reste d'eau.

3